# EUROPEAN PATENT APPLICATION

(11) **EP 1 797 924 A1**
(43) Date of publication of application: **20.06.2007**
(21) Application number: 05027741.7
(22) Date of filing: 19.12.2005
(51) Int. Cl.: A61Q 19/10

(54) **Baby wipe kit**

(71) Applicant: BIONEST Ltd., Dublin 6 (IE)
(72) Inventor: Gobbi, Rosa Maria, 1950 Sion (CH)
(74) Representative: Minoja, Fabrizio

(57) **Abstract**

This invention relates to a kit containing wet wipes designed for babies, consisting of conventional baby wipes and wipes impregnated with colostrum, for use when changing nappies.

## Description

This invention relates to a kit containing wet wipes designed for babies.

More particularly, the invention relates to a kit consisting of conventional baby wipes and wipes impregnated with colostrum and/or sericin and possibly Vitamin E, for use when changing a baby's nappy.

The use of disposable nappies, which has been widespread in Western countries for decades, offers undeniable advantages in terms of practicality and convenience, but is also the main cause of a very common disorder: nappy rash.

In babies, the thin stratum corneum of the skin consists of a few layers of cells coated with a thin hydrolipid film, which constitutes a homogeneous acid layer (pH 4.2-5.6) that acts as a barrier against external agents. The sebaceous secretion that gives rise to the hydrolipid film is present at birth as a result of the action of the mother's hormones; however, this action disappears in a few months, and sebaceous production falls to very low levels. A baby's skin is therefore very delicate, and can easily be attacked by external agents of various origins, causing a greater predisposition to irritations and infections.

The barrier properties of the skin can be damaged or reduced by factors such as heat, hydration, variations in pH, the lipid concentration in the stratum corneum and chemical irritation caused by increased skin pH due to the ammonia produced by the action of bacterial enzymes on the urine and faecal proteases on the stools.

Moreover, the occlusive action exerted by the outer layer of disposable nappies, which consists of waterproof plastic, prevents the skin from breathing and causes an increase in temperature, permeability and humidity. These conditions constitute the ideal environment for bacterial colonisation. The mechanical effect exerted when the nappy rubs against the skin is an additional irritant factor.

Plastic pants, infrequent nappy changes and the use of aggressive cleansers that damage the delicate hydrolipid film can aggravate the situation. Another factor which can contribute to the onset of dermatitis is teething, which causes a temporary reduction in the immune defences, and often an increase in the acidity of the stools.

The consequence of this situation is napkin dermatitis (nappy rash), the most common skin disease among babies, which is manifested as a reddening of the perigenital area that comes into contact with faeces and urine. In the most serious forms, papules, blisters and cracks in the skin appear on the perineum, buttocks, lower abdomen and thighs. A frequent complication of nappy rash is supervening Candida albicans (thrush) in the affected area. Bacterial infection caused by staphylococci is more unusual.

In the mildest forms it is sufficient to ensure appropriate hygiene, apply a protective zinc oxide cream or paste, and leave the area affected by the rash uncovered by nappies as often as possible. Anti-inflammatory treatments are often used in the case of irritative lesions. If small pustules are observed, the rash is probably infected, and requires antiseptic treatment. In the case of supervening thrush, imidazole derivatives are often used.

To prevent nappy rash, it is useful to change the nappy frequently, cleanse with mild cleansers rather than soap, dry the affected part thoroughly, and apply a protective paste or cream to the skin to restore the hydrolipid film. The use of baby wipes, which were originally designed for wiping babies outside the home, has become increasingly popular, and has often replaced normal washing with water and a cleansing product even in the home. The mechanical action is combined with the chemico-physical action of the formulation and the absorbent medium. However, these wipes are not always formulated appropriately, and sometimes perform an excessive cleansing action. Moreover, when they are used outside the home, they cannot replace the application of protective creams and pastes; however, such creams and pastes present drawbacks when not used in an appropriate environment, because they make the hands of the person applying them to the child greasy or dirty, and can also dirty the surrounding environment if the child moves too much during application.

This invention is designed to overcome the problem described above, with the use of a baby wipe kit consisting of a first conventional wipe and a second wipe, to be used after the first, which is impregnated with colostrum and/or sericin and possibly Vitamin E.

Colostrum, the breast secretion observed from a few days before until a few days after birth, has a high immunoglobulin content. The moisturising, protective, anti-irritant, emollient, soothing, decongestant, healing action of colostrum has long been known, and colostrum is used to treat contact lesions and skin inflammations and disorders.

Sericin, a silk protein similar to keratin, is available in freeze-dried form and used in the cosmetic industry due to its skin moisturising and protective properties.

The use of wipes impregnated with colostrum and/or sericin after conventional wipes performs an emollient, soothing, anti-irritant, decongestant action on the baby's delicate skin, preventing the onset of inflammation and dermatitis, and helping to restore the normal condition of the epidermis in the case of existing dermatitis.

According to the invention, the wipes will be impregnated with mammal colostrum, preferably bovine colostrum, and/or sericin, in quantities sufficient to exert the desired soothing and protective action. The simultaneous presence of colostrum and sericin is particularly preferred.

Vitamin E can also help, due to its nutrient, emollient and antioxidant effects.

More particularly, according to the invention the cleansing wipes will consist of wipes made of non-woven fabric (NWF) impregnated with 4-5 ml of cleanser, while the wipes impregnated with colostrum will consist of wipes made of non-woven fabric impregnated with 4-5 ml of colostrum and/or an aqueous solution containing 1 to 10% of sericin.

Vitamin E (or derivatives thereof) can be added to the wipes by simple immersion, or can be sprayed onto them.

Preferably, according to the invention, the wipes will be constituted by 50 g/sq.m of NWF in the format 150 x 200 mm.

Preferably, according to the invention, the cleansing wipes will contain conventional cleansers, preferably based on demineralised water, glycerin, sorbitan stearate, polysorbate 60 and cetearyl alcohol.

Moreover, according to the invention, the cleansing wipes may also contain extracts of plant origin which are useful for the treatment of skin disorders, such as extracts of Cocos nucifera, Ricinus communis, Elaeis guineensis, Citrus medica, Calendula officinalis, Helycrysum arenarium, Citrus grandis, Citrus dulcis and Citrus nobilis.

The wipes according to the invention could contain other active constituents with a complementary or otherwise useful activity, such as anti-inflammatory, antibacterial, moisturising, emollient, wound-healing and skin-repair agents, together with excipients conventionally used in the preparation of wet wipes.

According to the invention, the kit will be constituted by conventional wet wipes and wipes impregnated with colostrum and/or sericin and possibly Vitamin E, contained in separate or double bonded polyester/aluminium/polythene sachets with an inner subdivision that separates the two different types of wipe, or in separate or single containers with a double opening, subdivided internally by a partition that forms two separate chambers, one for each type of wipe.

## Claims

1. Baby wipe kit designed for use when changing nappies, consisting of conventional baby wipes and wipes impregnated with colostrum and/or sericin and possibly Vitamin E.

2. Kit as claimed in claim 1, wherein the conventional cleansing wipes and the wipes impregnated with colostrum and/or sericin and possibly Vitamin E are presented in separate or double bonded polyester/aluminium/polythene sachets with an inner subdivision that separates the two different types of wipe, or in separate containers or containers with a double opening, subdivided internally by a partition that forms two separate chambers, one for each type of wipe.

3. Kit as claimed in claims 1 and 2, wherein the cleansing wipes consist of wipes made of non-woven fabric impregnated with 4-5 ml of cleanser, and the wipes impregnated with colostrum and/or sericin consist of wipes made of non-woven fabric impregnated with 4-5 ml of colostrum and/or an aqueous solution containing 1 to 10% of sericin.

4. Kit as claimed in claim 3, wherein the cleansing wipes are impregnated with a conventional cleanser, preferably based on demineralised water, glycerin, sorbitan stearate, polysorbate 60 and cetearyl alcohol, together with excipients conventionally used in the preparation of wet wipes.

5. Kit as claimed in claim 3, wherein the cleansing wipes also contain extracts of plant origin which are useful for the treatment of skin disorders, chosen from among extracts of *Cocos nucifera, Ricinus communis, Elaeis guineensis, Citrus medica, Calendula officinalis, Helycrysum arenarium, Citrus grandis, Citrus dulcis and Citrus nobilis.*

6. Kit as claimed in claim 3, wherein the cleansing wipes also contain other active constituents with a complementary or otherwise useful activity, such as anti-inflammatory, antibacterial, moisturising, emollient, wound-healing and skin-repair agents.
